# EUROPEAN PATENT SPECIFICATION

(11) **EP 2 010 013 B1**
(45) Date of publication and mention of the grant of the patent: **24.06.2009**
(21) Application number: 07727284.7
(22) Date of filing: 23.03.2007
(51) Int. Cl.: A23L 2/02, A23L 2/38, A23L 1/304, A23L 2/60, A23L 2/385, A23L 2/39

(54) **OXIDATION-STABLE GRANULATE CONTAINING UNSATURATED FATTY ACIDS**
OXIDATIONSTABILES GRANULAT, DAS UNGESÄTTIGTE FETTSÄUREN ENTHÄLT
GRANULE STABLE CONTRE L'OXYDATION CONTENANT DES ACIDES GRAS INSATURES

(30) Priority: 21.04.2006 EP 06075938
(43) Date of publication of application: 07.01.2009
(73) Proprietor: Unilever N.V., 3013 AL Rotterdam (NL); Unilever PLC, London EC4Y 0DY (GB)
(72) Inventor: CHÁVEZ MONTES, Bruno Edgar, 1012 Lausanne (CH); GREBENKÄMPER, Kai, 3133 AT Vlaardingen (NL); KOHLUS, Reinhard, 74074 Heilbronn (DE)
(74) Representative: Wurfbain, Gilles L.
(86) International application number: PCT/EP2007/052810
(87) International publication number: WO 2007/122053

(56) References cited:
- EP-B- 0 893 953
- WO-A-20/04047566
- WO-A-20/05006891
- US-B1- 6 521 247
- DATABASE WPI Week 199204 Derwent Publications Ltd., London, GB; AN 1992-029666 XP002434357 & JP 03 277237 A (NISSHIN SEITO KK) 9 December 1991 (1991-12-09)
- DATABASE WPI Week 199532 Derwent Publications Ltd., London, GB; AN 1995-242720 XP002434358 & JP 07 147932 A (TERUMO CORP) 13 June 1995 (1995-06-13)

## Description

### TECHNICAL FIELD OF THE INVENTION

The present invention relates to a water-dispersible granulate comprising 40-90 wt.% of carbohydrates, 2-30 wt.% of lipids and at least 3% of unsaturated fatty acids by weight of the granulate.

### BACKGROUND OF THE INVENTION

It is well-known in the food industry that the application of oils containing high levels of unsaturated fatty acids can give rise to serious off-flavour problems. These off-flavour problems are associated with the oxidation of the unsaturated fatty acids, leading to the formation of volatile, potent flavour molecules, such as unsaturated aldehydes. Flavour attributes associated with oxidation products of unsaturated fatty acids include "cardboard", "paint", "oily", "rancid", "metallic" and "fish".
Whilst all unsaturated fatty acids have been associated with the aforementioned stability problems, lipids containing polyunsaturated fatty acids, notably lipids containing ω-3 and/or ω-6 polyunsaturated fatty acids (PUFA) are known to be extremely susceptible to oxidation. Oxidation of the latter fatty acids yields volatile oxidation products that introduce a repugnant flavour to the product containing these fatty acid residues. Typical examples of ω-3 PUFA include docosahexaenoic acid (DHA), eicosapentaenoic acid (EPA) and α-linolenic acid (ALA). Typical examples of ω-6 fatty acid include linoleic acid (LA), γ-linolenic acid (GLA) and arachidonic acid (AA).

Although it is known that the rate of oxidation of unsaturated fatty acids may be reduced through the addition of anti-oxidants, the application of such anti-oxidants at best delays the formation of unacceptable off-flavour. However, the shelf-life of products containing appreciable amounts of unsaturated fatty acids, especially food products and beverages, is inherently limited as a result of the inevitable oxidation of these fatty acid residues.

Furthermore, in some product applications, e.g. in reconstitutable powders, it is extremely difficult to produce bland tasting products if these products contain a significant quantity of unsaturated fatty acids. The production of reconstitutable powders typically involves one or more processing steps at elevated temperatures (e.g. drying). The oxidation rate of unsaturated fatty acids, however, increases exponentially with incrementing temperature, especially if these fatty acids are exposed to elevated temperatures in the presence of air and/or pro-oxidants.

Many scientific publications have been issued that strongly suggest that regular consumption of significant amounts of polyunsaturated fatty acids can deliver important health benefits. Furthermore, there is ample evidence showing that in general unsaturated fatty acids are more healthy than saturated fatty acids. Hence, efforts have been made by the industry to develop food products and nutritional preparations that contain appreciable amounts of unsaturated fatty acids, especially polyunsaturated fatty acids. It has also been attempted to incorporate (poly)unsaturated fatty acids in reconstitutable powders.

US 2005/018019 describes the encapsulation of a polyunsaturated fatty acids, such as ω-3 and/or ω-6 PUFA, by spray drying an emulsion containing a carrier to form a powder, followed by encapsulation in a fluid bed dryer. Suitable carriers are said to include modified food starches, maltodextrins, proteins, sugars and mixtures thereof. It is stated in the US application that it is anticipated that the spray dried and encapsulated oils will be less susceptible to oxidation and the off tastes which accompany oxidation will be reduced. Example 2 describes how 1 kg of water, 100 g milk protein, 50 g modified food starch, 50 g flow starch and 200 g oil are emulsified using a high pressure homogeniser, followed by spray drying. The spray dried powder is introduced in a fluid bed coater and sprayed under a nitrogen blanket with a mixture of carnauba wax and paraffin.

US 2004/0201116 describes a process of producing oil containing pellets wherein the oil is protected against oxidation. Example 8 describes the preparation of pellets containing polyunsaturated oil by mixing 0.5 kg soybean-based cellulose with 2 kg maltodextrin and slowly dissolving the mixture in 12.5 kg demineralised water. Next, 7.5 kg of the same quality maltodextrin was added slowly together with 3 kg polyunsaturated oil with a high speed mixer to form 22.5 kg pre-emulsion which was homogenised with a two stage homogeniser at a pressure of 250 bar and 275 bar. The mixture was fed to a fluid bed direct pelletising pilot installation to produce pellets with a particle size of between 250-355 µm. Using the same ingredients and process also pellets of a particle size of between 800-1180 µm were prepared. All the pellets prepared had an oil content of more than 23%.

The aforementioned processes for producing unsaturated fatty acids containing powders suffer from a number of disadvantages. First of all, these processes are very elaborate, amongst others because they require the use of a substantial amount of water to prepare a pre-emulsion, whereas the same water needs to be removed later to yield a powder. Furthermore, the processes described in the prior art employ a spray drying step which, as mentioned herein before, due to the elevated temperatures accelerates oxidation of the unsaturated fatty acids. Finally, powders having a small particle size, such as those described in US 2005/0181019 suffer from the disadvantage that they are not readily water-dispersible.

EP-A 0 893 953 describes solid carrier particles onto whose outer surface has been coated or absorbed at least one polyunsaturated fatty acid in liquid form, the particles being suitable for consumption by humans, and dispersible or miscible in water. Example 5 describes the preparation of a PUFA containing powder by mixing 75% maltodextrin (Glucidex® 19) with 25% fungal arachidonic oil (40% ARA) in a high speed mixer (grall® 300, Colette; 100 kg; mixing for 10 minutes, speed 1 for mixing arm and chopper). The maltodextrin Glucidex® 19 has a DE of 19 and a particle size well below 200 µm.

Also in other areas, such as the flavour industry, efforts have been made to prepare oxidation-stable particulate compositions containing oxidation sensitive flavouring materials. US 5,506,353 describes the use of maltodextrin having a low DE in the preparation of a particulate flavour composition comprising a flavour oil (e.g. citrus oil). Example 3 describes the preparation of a particulate flavour composition comprising orange oil. The composition was prepared by centrifugal atomisation of an emulsion containing 43.61 wt.% water; 7.93 wt.% orange oil; 35.25 wt.% Hystar®; 9.25 wt.% Maltrin® M040 and 3.96 wt.% Capsul®. It is observed that the resulting particulate flavour composition showed no oxidation after having been stored at 60°C for 4 weeks. Maltrin® M040 is a maltodextrin having 5 DE. The average particle size of the particulate composition appears to be well below 200 µm.

US-B-6 521 247 discloses a nutritional supplement for a human, said supplement comprising folic acid, a first pharmaceutically acceptable iron compound selected to be slowly dissolving and a second, different pharmaceutically acceptable iron compound selected to be rapidly dissolving.
WO-A-2004/047566 discloses nutritional compositions for liver disease patients as well as nutritional compositions for patients under high levels of invasive stress. JP-A-03 277 237 discloses powdered sugar compositions for sprinkling on products such as bread and cakes.

There is compelling scientific evidence suggesting that iron deficiency during childhood hamper mental development.
Furthermore, it has been suggested that cognitive capabilities may be impaired as a result of iron deficiency. Hence, it is desirable to supplement nutritional products with iron. From a nutritional perspective, also supplementation with copper is desirable. Both iron and copper are well-known for their pro-oxidative properties. This is why product developers will always try to minimize iron and copper levels in foodstuffs and beverages that contain unsaturated lipids. Hence, it is a major challenge to provide an oxidation-stable granulate comprising a substantial amount of unsaturated fatty acids as well as a significant amount of iron and/or copper.

### SUMMARY OF THE INVENTION

The inventors have succeeded in developing a readily water-dispersible granulate that contains a substantial amount of unsaturated fatty acids as well as a high level of iron and/or copper and which granulate is nonetheless very stable against oxidation. The granulate of the present invention is easy to manufacture by a process that does not employ emulsification or pro-oxidative conditions.

The inventors have found that water-dispersible, highly oxidation-stable granulates comprising (i) 2-30 wt.% of lipids containing at least 3% of unsaturated fatty acids by weight of the granulate and (ii) at least 30 mg/kg of pro-oxidative metal in the form of iron and/or copper can be produced without difficulty by:
• incorporating the lipids containing the unsaturated fatty acids as free or non-dispersed lipids;
• incorporating carbohydrates in an amount of 40-90% by weight of the granulate; and
• ensuring that the mass weighted average diameter of the granulate exceeds 100 µm.

The present granulate can be produced under very mild conditions by simply absorbing the lipids onto granules containing the carbohydrates and the pro-oxidative metal. Unlike spray drying methods, this method does not yield a powder in which the lipids containing the unsaturated fatty acids are dispersed in a carbohydrate matrix that protects the lipids from the surrounding atmosphere. In the present granulate, the lipids are present as free oil, rendering it truly surprising that the granulate exhibits exceptional oxidation stability in the presence of pro-oxidative metal.

### DETAILED DESCRIPTION OF THE INVENTION

Thus, one aspect of the invention relates to a water-dispersible granulate having a mass weighted average diameter of at least 100 µm, preferably of at least 200 µm, said granulate comprising:
• 40-90 wt.% of carbohydrates;
• 2-30 wt.% of lipids containing at least 1% unsaturated fatty acids by weight of the granulate and
• at least 30 mg/kg of a pro-oxidative metal selected from the group consisting of iron, copper and combinations thereof; wherein at least 50wt.%, preferably at least 70wt.% of the lipids is present as non-dispersed lipids.

The terms "mean diameter" and "average diameter" whenever used herein, refer to the mass-weighted average diameter. The mass-weighted average diameter of a granulate is suitably determined with the help of sieves.

The term "lipid" as used herein encompasses fatty acid containing lipids selected from the group consisting of triglycerides, diglycerides, monoglycerides, phospholipids, free fatty acids and combinations thereof. Most preferably, the lipids of the present invention are selected from the group consisting of triglycerides, diglycerides and combinations thereof. Most preferably, the lipids are triglycerides. The term "oil" as used herein encompasses both lipids that are liquid or solid at e.g. 20°C. Preferably, the lipids have a melting point of less than 40°C, more preferably of less than 30°C, even more preferably of less than 20°C and most preferably of less than 10°C.

The term "fatty acid" as used herein encompasses free fatty acids as well as fatty acid residues. Thus, the unsaturated fatty acids of the present invention may be contained in the granulate in the form of fatty acids residues of e.g. triglycerides and/or as free fatty acids.

The term "water dispersible" as used herein means that a granulate passes the following test: A sample of 20 g is added to a glass beaker (diameter=6 cm) containing 140 ml of water having a temperature of 75 °C. The mixture is stirred with a magnetic stirrer at a speed that produces a vortex in the liquid with a depth of 2 mm. Immediately thereafter, the stirred mixture is poured over a 250 µm sieve (diameter=10 cm). After 1 minute, the sieve is dried in an oven at 110 °C for 2 hours and weighted. If the observed weight increase of the sieve is less than 1 g, the granulate is water-dispersible. If not, the granulate is not water-dispersible. According to a particularly preferred embodiment, the granulate is cold water dispersible, meaning that the powder is dispersible under the aforementioned test conditions, employing water with a temperature of 10°C.

An important advantage of the present granulate resides in the fact that it can be produced without the preparation of a pre-emulsion in which the lipids are dispersed in a continuous (aqueous) phase containing one or more hydrocolloids. The use of such a pre-emulsion ensures that during drying, the dispersed lipids become encased as a dispersed phase in a matrix that is made up of the one or more hydrocolloids. In contrast, in the present granulate, the lipids are suitably absorbed onto pre-formed particles containing the carbohydrates and the pro-oxidative metal. Thus, in the granulate of the present invention, a substantial amount of the lipids is present as non-dispersed oil. Preferably, at least 80 wt.%, most preferably, at least 90 wt.% of the lipids is present as non-dispersed oil. The amount of lipids that is present as non-dispersed oil can suitably be determined by measuring the amount of "free fat", using the methodology described by Vega et al., J. Food Sci. (2005), 30:144-251.

The granulates made from pre-emulsions typically contain droplets of dispersed oil that have a small average diameter that is typically within the range of 0.1-10 µm. In contrast, in the present granulate preferably less than 30 wt.%, more preferably less than 10 wt.% and most preferably less than 5 wt.% of the lipids is present as dispersed oil droplets with a diameter of less than 5 µm, especially of less than 10 µm.

The benefits of the present invention are particularly pronounced in case the granulates contains at least 3 wt.% of polyunsaturated fatty acids (PUFA), especially polyunsaturated acids selected from the group consisting of ω-3 fatty acids, ω-6 fatty acids and combinations thereof.

The granulates of the present invention are very stable against oxidation, even if these granulates contain substantial levels of the pro-oxidant metal. According to a particularly preferred embodiment, the granulate contains at least 60 mg/kg, more preferably at least 120 mg/kg and most preferably at least 200 mg/kg of the pro-oxidative metal. Usually, the content of the pro-oxidative metal does not exceed 800 mg/kg. According to a particularly preferred embodiment, the pro-oxidative metal is iron. The benefits of the present invention are particularly pronounced in case the iron is present as ferrous and/or ferric iron. Most preferably, the iron is present as ferrous iron.

Iron is suitably incorporated in the present granulates in the form of a water-soluble or water-insoluble salt. Examples of iron salt that may advantageously be incorporated in the granulate include: iron fumarate, iron sulphate, iron EDTA, haeme iron, iron phosphate, iron pyrophosphate and iron citrate. According to a preferred embodiment, iron is incorporated as in the form of one or more iron salts selected from the group consisting of iron fumarate, iron sulphate, iron EDTA and haeme iron. The latter iron salts offer the advantage that they exhibit good bioavailability. Most preferably, the iron is incorporated in the form of iron fumarate.

The inventors have discovered that the granulates exhibiting particularly good oxidative stability can be prepared by incorporating 20-55 wt.% of maltodextrin, preferably with a DE value comprised between 2 and 20.

According to a particularly preferred embodiment, the present granulate comprises at least 4 wt.%, more preferably 6-25 wt.%, most preferably 9-20 wt.% of lipids. Surprisingly, it was found that the present granulate retains both excellent flowability and oxidation stability even if it contains up to 20 wt.% lipids.

The PUFA present in granulate of the invention are advantageously contained as fatty acids residues in the lipids. Typically, the lipids contain at least 20 wt.%, preferably at least 30 wt.% and most preferably at least 50 wt.% of unsaturated fatty acid residues, said percentages being calculated on the total amount of fatty acid residues contained in the lipids. Preferably, said unsaturated fatty acids are polyunsaturated fatty acids, most preferably polyunsaturated fatty acids selected from the group consisting of ω-3 fatty acids, ω-6 fatty acids and combinations thereof.

Suitable sources of ω-3 fatty acids and ω-6 fatty acids include fish oil, algae oil, linseed oil, rapeseed oil, soybean oil, sunflower oil and corn oil, fish oil, algae oil, linseed oil and rapeseed oil being particularly preferred. The benefits of the present invention are particularly apparent if the granulate contains at least 0.2 wt.%, preferably at least 0.4 wt.% of a triglyceride oil selected from the group consisting of fish oil, algae oil, linseed oil and combinations thereof.

Polyunsaturated acids that are particularly sensitive to oxidation and that produce particularly repugnant oxidation products include linoleic acid, α-linolenic acid, eicosapentaenoic acid, docosahexaenoic acid and arachidonic acid. Hence, according to a particularly preferred embodiment, the present granulate comprises at least 0.1 wt.%, preferably at least 0.5 wt.%, more preferably at least 1 wt.% and most preferably at least 3 wt.% of polyunsaturated acids selected from the group consisting of linoleic acid, α-linolenic acid, eicosapentaenoic acid, docosahexaenoic acid, arachidonic acid and combinations thereof.

Besides maltodextrin and the lipids, the present granulate may suitably contain other components, especially hydrophilic components such as carbohydrates, protein, salts and acids. Typically, the present granulate contains at least 50 wt.%, more preferably at least 70 wt.% of carbohydrates. Here the term carbohydrates encompasses any material that predominantly, preferably exclusively consists of monosaccharides and/or polymers of such monosaccharide units. Examples of carbohydrates include monosaccharides (e.g. glucose, fructose), disaccharides (e.g. sucrose), trisaccharides (e.g. maltriose), oligosaccharides (e.g. high DE maltodextrin) and polysaccharides (e.g starch and low DE maltodextrin).

According to a very preferred embodiment, the granulate comprises between 20 and 65 wt.%, preferably between 25 and 55 wt.% of a saccharide selected from the group consisting of monosaccharides, disaccharides and combinations thereof.

The mean particle diameter of the present granulate usually does not exceed 2000 µm. Water dispersibility of the present granulate is believed to be optimal if the granulate has a mean particle diameter of more than 400 µm and not more than 1500 µm. Most preferably, the mean particle diameter is within the range of 600-1200 µm.

In order to be able to absorb a significant quantity of lipids, the non-lipid matrix of the present granulate needs to be porous. Accordingly, in a preferred embodiment, the present granulate has a bulk density within the range of 300-600 g/l, more preferably within the range of 350-450 g/l.

The present granulate, besides unsaturated fatty acids advantageously contains a number of nutritionally desirable ingredients such as minerals, vitamins, flavonoids, carotenoids, etc. In an advantageous embodiment, the granulate comprises between 1 and 20 g/kg, preferably between 2 and 10 g/kg of vitamin C. Also oxidation of vitamin C can be prevented very effectively by incorporating vitamin C in the present granulate.

In another preferred embodiment, the granulate comprises between 10 and 100 mg/kg, preferably between 30 and 80 mg/kg of vitamin E. The inclusion of vitamin E offers the advantage that it provides the PUFA with additional protection against oxidation.

The moisture content of the present granulate typically is less than 8 wt.%, preferably less than 5 wt.% moisture. The water activity (a_{w}) of the granulate is preferably between 0.1 and 0.4, more preferably between 0.15 and 0.3.

Another aspect of the invention relates to a process of preparing a granulate according to any one of the preceding claims, said process comprising the steps of:
• preparing core particles containing the carbohydrates and the pro-oxidative metal, said core particles having a mass-weighted average diameter of at least 100 µm;
• spraying the core particles with lipids containing the unsaturated fatty acids and allowing the lipids to be absorbed by the core particles; and
• recovering the granulate.

In accordance with a particularly preferred embodiment, the core particles are prepared by agglomerating a powder comprising the carbohydrates and the pro-oxidative metal and optionally other powder ingredients, by spraying the powder with an aqueous liquid. In an even more preferred embodiment, the powder is sprayed with an aqueous solution of a saccharide selected from the group consisting of monosaccharides, disaccharides and combinations thereof, followed by drying to obtain the agglomerated powder. The aqueous solution typically contains 5-35 wt.%, preferably 10-30 wt.% of saccharide.

In the present process the core particles are advantageously prepared by creating a fluid bed of the powder to be agglomerated and by spraying it with a liquid to achieve agglomeration. This advantageous embodiment of the invention may suitably be operated in a fluid bed agglomerating device.

As explained herein before, the core particles should have a certain level of porosity in order to enable the absorption of a substantial amount of lipids. Preferably, the bulk density of the core particles employed in the present method is within the range of 250-500 g/l, more preferably of 300-450 g/l.

A major advantage of the present invention lies in the fact that it can be operated under mild conditions, especially at the stage when the lipids are sprayed onto the core particles. Typically, the core particles are sprayed with the lipids at a temperature below 50°C, preferably below 40°C, most preferably at a temperature below 35°C.

As mentioned herein before, iron deficiency during childhood can hamper mental development and cognitive capabilities may be impaired as a result of such iron deficiency. Also polyunsaturated fatty acids, especially ω-3 fatty acids are believed to play an important role in mental development and cognitive function. The granulates according to the present invention are a particularly useful vehicle for delivering a combination of nutritional components that can be used in the treatment of prevention of retarded mental development or diminished cognitive function. Accordingly, another aspect of the invention relates to the use of the granulates of the present invention in the preparation of a composition for the treatment or prevention of retarded mental development or diminished cognitive function.

The invention is further illustrated by means of the following

### examples.

### EXAMPLES

### Example 1

A granulate according to the present invention was prepared using a fluid bed agglomerater (Aeromatic™ S-3, fluid bed granulator [Aeromatic-Fielder - GEA/NIRO]).

The fluid bed agglomerater was loaded with 21.5 kg of a powder mixture of the following composition:

| | Wt.% |
|---|---|
| Sugar | 38.9 |
| Maltodextrin DE 2 (Glucidex 2) | 25.4 |
| Maltodextrin DE 12 (Glucidex IT12) | 25,4 |
| Wheat flour | 8.5 |
| Vitamin/mineral/colours/fla vours blend ^{#} | 1.8 |
| | 100 |

| | |
|---|---|
| # Comprising micronised iron fumarate (ex Dr Paul Lohmann GmbH, Germany) and vitamin C | |

The fluid bed was heated to 42°C and sprayed with a 2700g of a 20% sugar solution at a rate of 255 g/min. Subsequently, the fluid bed consisting of agglomerated granules was dried for 10-12 minutes with air whilst maintaining a bed temperature of not more than 45°C. Next, the temperature of the fluid bed was reduced to 25°C, following which 3.5 kg of liquid oil was sprayed onto the fluidised granules at a rate of 250 g/min, whilst maintaining a bed temperature of not more than 30°C.

The liquid oil consisted of 25 parts soybean oil and 1 part fish oil (containing >32 wt.% DHA and <5.5 wt.% EPA).

A granulate was obtained with the following composition:

| | Wt.% |
|---|---|
| Maltodextrin DE 2 | 22 |
| Maltodextrin DE 12 | 22 |
| Wheat flour | 7.3 |
| Sugar | 34.3 |
| Soya oil ^{$} | 12.2 |
| Fish oil ^{&} | 0.5 |
| Vitamin/mineral mix/colours/flavours ^{#} | 1.6 |
| | 100 |

| | |
|---|---|
| # Including 300 mg/kg iron and 6 g/kg vitamin C by weight of total granulate & Containing 0.8 mg/g α-tocopherol (vitamin E) $ Containing 3 mg/g α-tocopherol | |

The granulate so obtained was water-dispersible, had a mass weighted mean particle diameter of 600 µm and a bulk density of 380 g/l.

Part of the granulate was packaged under vacuum in sachets (21 g per sachet), which sachets were sealed by an inner layer of aluminium and PP with a thickness 9 µm. Another part of the granulate was packaged in sachets (21 g per sachet) made of BOPP (biaxially oriented polypropylene) film. The aluminum sachets were stored for 3 months at 40°C and a relative humidity of 90%. The BOPP sachets were stored for 1 month under the same conditions. After these storage periods, the granules were dissolved into 140 ml of cold milk and the resulting beverages were tasted by an expert panel. The panel members concluded that the quality of the beverages was acceptable. More particularly, they found that the beverages did not contain objectionable off-flavours resulting from oil oxidation.

### Example 2

Example 1 was repeated except that this time the fluid bed agglomerater was loaded with a powder mixture of the following composition:

| | Wt.% |
|---|---|
| Maltodextrin DE 12(Maldex 120, ex Tate and Lyle) | 47.42 |
| Powder sugar | 40.84 |
| Wheat flour | 9.48 |
| Vitamin/mineral/colours/flavours blend ^{#} | 2.26 |
| | 100 |

| | |
|---|---|
| # Comprising micronised iron fumarate (ex Dr Paul Lohmann GmbH, Germany) and vitamin C | |

The liquid that was sprayed onto the fluidised granules consisted of 25 parts soybean oil and 1 part fish oil (containing 16 wt.% DHA and 32 wt.% EPA). The final granulate had the following composition:

| | Wt.% |
|---|---|
| Maltodextrin DE 12 | 38.51 |
| Powder sugar | 35.94 |
| Soybean oil^{$} | 15.40 |
| Fish oil^{&} | 0.62 |
| Wheat flour | 7.70 |
| Vitamin/mineral/colours/flavours blend | 1.83 |
| | 100 |

| | |
|---|---|
| # Including 314 mg/kg iron and 6.28 g/kg vitamin C by weight of total granulate & Containing 0.8 mg/g α-tocopherol (vitamin E) $ Containing 3 mg/g α-tocopherol | |

Storage trials produced the same results as described in Example 1.

### Example 3

Example 1 was repeated except that this time the fluid bed agglomerater was loaded with a powder mixture of the following composition:

| | Wt.% |
|---|---|
| Maltodextrin DE 12 (Glucidex IT 12, ex Roquette) | 25.6 |
| Maltodextrin DE 12 (Maldex 120, ex Tate and Lyle) | 25.6 |
| Powder sugar | 36.8 |
| Wheat flour | 8.53 |
| Vitamin/mineral/colours/flavours blend ^{#} | 3.51 |
| | 100 |

| | |
|---|---|
| # Comprising micronised iron fumarate (ex Dr Paul Lohmann GmbH, Germany) and vitamin C | |

The liquid that was sprayed onto the fluidised granules consisted of 100 parts soybean oil and 3.6 parts fish oil. The final granulate had the following composition:

| | Wt.% |
|---|---|
| Maltodextrin DE 12 | 43.4 |
| Powder sugar | 33.8 |
| Soybean oil^{$} | 12.1 |
| Fish oil^{&} | 0.43 |
| Wheat flour | 7.24 |
| Vitamin/mineral/colours/flavours blend | 2.98 |
| | 100 |

| | |
|---|---|
| # Including 296 mg/kg iron and 5.91 g/kg vitamin C by weight of total granulate & Containing 0.8 mg/g α-tocopherol $ Containing maximum 4.6 mg/g α-tocopherol | |

Storage trials produced the same results as described in Example 1.

## Claims

1. A water-dispersible granulate having a mass weighted average diameter of at least 100 µm, preferably of at least 200 µm, said granulate comprising:
• 40-90 wt.% of carbohydrates;
• 2-30 wt.% of lipids containing at least 1%, preferably at least 3% of unsaturated fatty acids by weight of the granulate and
• at least 30 mg/kg of a pro-oxidative metal selected from the group consisting of iron, copper and combinations thereof;
wherein at least 50 wt.%, preferably at least 80 wt.% of the lipids is present as non-dispersed lipids.

2. Granulate according to claim 1, wherein the granulate contains at least 1 wt.%, preferably at least 3 wt.% of polyunsaturated fatty acids, preferably polyunsaturated fatty acids selected from the group consisting of ω-3 fatty acids, ω-6 fatty acids and combinations thereof.

3. Granulate according to claim 1 or 2, wherein the granulate contains at least 60 mg/kg, preferably at least 120 mg/kg of the pro-oxidative metal.

4. Granulate according to any one of the preceding claims, wherein the pro-oxidative metal is iron.

5. Granulate according to any one of the preceding claims, wherein the granulate contains 20-55 wt.% of maltodextrin.

6. Granulate according to any one of the preceding claims, wherein the granulate comprises between 20 and 65 wt.% of a saccharide selected from the group consisting of monosaccharides, disaccharides and combinations thereof.

7. Granulate according to any one of the preceding claims, wherein less than 30 wt.%, preferably less than 10 wt.% of the lipids is present as dispersed droplets with a diameter of less than 5 µm.

8. Granulate according to any one of the preceding claims, wherein the pro-oxidative metal is present within the granules of the granulate in concentration spots.

9. Granulate according to any one of the preceding claims, wherein the granulate comprises 5-25 wt.%, preferably 9-20 wt.% of lipids.

10. Granulate according to any one of the preceding claims, wherein the lipids are selected from the group consisting of triglycerides, diglycerides, monoglycerides, phosphoglycerides, free fatty acids and combinations thereof.

11. Granulate according to any one of the preceding claims, wherein the lipids contain at least 50 wt.% of polyunsaturated fatty acid residues selected from the group consisting of ω-3 fatty acids, ω-6 fatty acids and combinations thereof.

12. Granulate according to any one of the preceding claims, wherein the granulate has a mass weighted mean particle diameter of more than 400 µm and not more than 1500 µm.

13. Granulate according to any one of the preceding claims, wherein the granulate has a bulk density within the range of 300-600 g/l, preferably within the range of 350-450 g/l.

14. A process of preparing a granulate according to any one of the preceding claims, said process comprising the steps of:
• preparing core particles containing the carbohydrates and the pro-oxidative metal, said core particles having a mass weighted average diameter of at least 100 µm;
• spraying the core particles with the lipids containing the unsaturated fatty acids and allowing the lipids and the unsaturated acids to be absorbed by the core particles; and
• recovering the granulate.

15. Process according to claim 14, wherein the core particles are prepared by agglomerating a powder comprising the pro-oxidative metal and optionally other powder ingredients, by spraying the powder with an aqueous liquid.

16. Process according to claim 15, wherein the powder is sprayed with an aqueous solution of a saccharide selected from the group consisting of monosaccharides, disaccharides and combinations thereof, followed by drying to obtain the agglomerated powder.

17. Process according to any one of claims 14-16, wherein the core particles are sprayed with the lipids at a temperature below 50°C, preferably below 40°C.

18. Use of a granulate according to any one of claims 1-13 in the preparation of a composition for use in the treatment or prevention of retarded mental development or diminished cognitive function.

## Patentansprüche

1. In Wasser dispergierbares Granulat, das einen massengewichteten mittleren Durchmesser von wenigstens 100 µm, vorzugsweise wenigstens 200 µm hat, wobei das Granulat umfasst:
• 40-90 Gewichts-% Kohlenhydrate;
• 2-30 Gewichts-% Lipide, die wenigstens 1 Gewichts-%, vorzugsweise wenigstens 3 Gewichts-% ungesättigte Fettsäuren, bezogen auf das Granulat, enthalten, und
• wenigstens 30 mg/kg eines pro-oxidativen Metalls, ausgewählt aus der Gruppe, bestehend aus Eisen, Kupfer und Kombinationen davon,
wobei wenigstens 50 Gewichts-%, vorzugsweise wenigstens 80 Gewichts-% der Lipide als nicht-dispergierte Lipide vorliegen.

2. Granulat gemäß Anspruch 1, wobei das Granulat wenigstens 1 Gewichts-%, vorzugsweise wenigstens 3 Gewichts-%, mehrfach ungesättigte Fettsäuren, vorzugsweise mehrfach ungesättigte Fettsäuren, ausgewählt aus der Gruppe, bestehend aus ω-3-Fettsäuren, ω-6-Fettsäuren und Kombinationen davon, enthält.

3. Granulat gemäß Anspruch 1 oder 2, wobei das Granulat wenigstens 60 mg/kg, vorzugsweise wenigstens 120 mg/kg des pro-oxidativen Metalls enthält.

4. Granulat gemäß einem der vorangehenden Ansprüche, wobei das pro-oxidative Metall Eisen ist.

5. Granulat gemäß einem der vorangehenden Ansprüche, wobei das Granulat 20-55 Gewichts-% Maltodextrin enthält.

6. Granulat gemäß einem der vorangehenden Ansprüche, wobei das Granulat zwischen 20 und 65 Gewichts-% eines Saccharids, ausgewählt aus der Gruppe, bestehend aus Monosacchariden, Disacchariden und Kombinationen davon, umfasst.

7. Granulat gemäß einem der vorangehenden Ansprüche, wobei weniger als 30 Gewichts-%, vorzugsweise weniger als 10 Gewichts-% der Lipide als dispergierte Tröpfchen mit einem Durchmesser von weniger als 5 µm vorliegen.

8. Granulat gemäß einem der vorangehenden Ansprüche, wobei das pro-oxidative Metall innerhalb der Körnchen des Granulats in Konzentrationsflecken vorliegt.

9. Granulat gemäß einem der vorangehenden Ansprüche, wobei das Granulat 5-25 Gewichts-%, vorzugsweise 9-20 Gewichts-% Lipide umfasst.

10. Granulat gemäß einem der vorangehenden Ansprüche, wobei die Lipide aus der Gruppe, bestehend aus Triglyceriden, Diglyceriden, Monoglyceriden, Phosphoglyceriden, freien Fettsäuren und Kombinationen davon, ausgewählt sind.

11. Granulat gemäß einem der vorangehenden Ansprüche, wobei die Lipide wenigstens 50 Gewichts-% mehrfach ungesättigte Fettsäurereste, ausgewählt aus der Gruppe, bestehend aus ω-3-Fettsäuren, ω-6-Fettsäuren und Kombinationen davon, enthalten.

12. Granulat gemäß einem der vorangehenden Ansprüche, wobei das Granulat einen massengewichteten mittleren Partikeldurchmesser von mehr als 400 µm und nicht mehr als 1500 µm hat.

13. Granulat gemäß einem der vorangehenden Ansprüche, wobei das Granulat eine Schüttdichte im Bereich von 300-600 g/l, vorzugsweise im Bereich von 350-450 g/l, hat.

14. Verfahren zur Herstellung eines Granulats gemäß einem der vorangehenden Ansprüche, wobei das Verfahren die Schritte:
• Herstellen von Kernpartikeln, die die Kohlenhydrate und das pro-oxidative Metall enthalten, wobei die Kernpartikel einen massengewichteten mittleren Durchmesser von wenigstens 100 µm haben;
• Besprühen der Kernpartikel mit den Lipiden, die die ungesättigten Fettsäuren enthalten, und Absorbierenlassen der Lipide und der ungesättigten Säuren durch die Kernpartikel und
• Gewinnen des Granulats
umfasst.

15. Verfahren gemäß Anspruch 14, wobei die Kernpartikel durch Agglomerieren eines Pulvers, das das pro-oxidative Metall und gegebenenfalls andere Pulverinhaltsstoffe umfasst, durch Besprühen des Pulvers mit einer wässrigen Flüssigkeit hergestellt werden.

16. Verfahren gemäß Anspruch 15, wobei das Pulver mit einer wässrigen Lösung eines Saccharids, ausgewählt aus der Gruppe, bestehend aus Monosacchariden, Disacchariden und Kombinationen davon, besprüht wird, gefolgt von einem Trocknen unter Erhalt des agglomerierten Pulvers.

17. Verfahren gemäß einem der Ansprüche 14-16, wobei die Kernpartikel mit den Lipiden bei einer Temperatur unter 50 °C, vorzugsweise unter 40 °C, besprüht werden.

18. Verwendung eines Granulats gemäß einem der Ansprüche 1-13 bei der Herstellung einer Zusammensetzung zur Verwendung bei der Behandlung oder Prävention einer verzögerten mentalen Entwicklung oder einer verminderten kognitiven Funktion.

## Revendications

1. Granulé dispersible dans l'eau possédant un diamètre moyen pondéré d'au moins 100 µm, de préférence d'au moins 200 µm, ledit granulé comprenant :
- 40 à 90 % en poids de glucides ;
- 2 à 30 % en poids de lipides contenant au moins 1 %, de préférence au moins 3 % d'acides gras insaturés en poids du granulé, et
- au moins 30 mg/kg d'un métal pro-oxydant choisi dans le groupe constitué par le fer, le cuivre et leurs combinaisons ;
dans lequel au moins 50 % en poids, de préférence au moins 80 % en poids des lipides sont présents sous forme de lipides non dispersés.

2. Granulé selon la revendication 1, dans lequel le granulé contient au moins 1 % en poids, de préférence au moins 3 % en poids d'acides gras polyinsaturés, de préférence des acides gras polyinsaturés choisis dans le groupe constitué par les acides gras ω-3, les acides gras ω-6 et leurs combinaisons.

3. Granulé selon la revendication 1 ou 2, dans lequel le granulé contient au moins 60 mg/kg, de préférence au moins 120 mg/kg du métal pro-oxydant.

4. Granulé selon l'une quelconque des revendications précédentes, dans lequel le métal pro-oxydant est le fer.

5. Granulé selon l'une quelconque des revendications précédentes, dans lequel le granulé contient 20 % à 55 % en poids de maltodextrine.

6. Granulé selon l'une quelconque des revendications précédentes, dans lequel le granulé comprend entre 20 et 65 % en poids d'un saccharide choisi dans le groupe constitué par les monosaccharides, les disaccharides et leurs combinaisons.

7. Granulé selon l'une quelconque des revendications précédentes, dans lequel moins de 30 % en poids, de préférence au moins de 10 % en poids des lipides sont présents sous forme de gouttelettes dispersées ayant un diamètre inférieur à 5 µm.

8. Granulé selon l'une quelconque des revendications précédentes, dans lequel le métal pro-oxydant est présent dans les granules du granulé en points de concentration.

9. Granulé selon l'une quelconque des revendications précédentes, dans lequel le granulé comprend 5 à 25 % en poids, de préférence 9 à 20 % en poids de lipides.

10. Granulé selon l'une quelconque des revendications précédentes, dans lequel les lipides sont choisis dans le groupe constitué par les triglycérides, les diglycérides, les monoglycérides, les phosphoglycérides, les acides gras libres et leurs combinaisons.

11. Granulé selon l'une quelconque des revendications précédentes, dans lequel les lipides contiennent au moins 50 % de résidus d'acides gras polyinsaturés choisis dans le groupe constitué par les acides gras ω-3, les acides gras ω-6 et leurs combinaisons.

12. Granulé selon l'une quelconque des revendications précédentes, dans lequel le granulé possède un diamètre moyen pondéré de particule supérieur à 400 µm et d'au plus 1 500 µm.

13. Granulé selon l'une quelconque des revendications précédentes, dans lequel le granulé possède une masse volumique apparente située dans la plage allant de 300 à 600 g/l, de préférence dans la plage allant de 350 à 450 g/l.

14. Procédé de préparation d'un granulé selon l'une quelconque des revendications précédentes, ledit procédé comprenant les étapes suivantes :
- la préparation de particules de coeur contenant les glucides et le métal pro-oxydant, lesdites particules de coeur possédant un diamètre moyen pondéré d'au moins 100 µm ;
- la pulvérisation des particules de coeur avec les lipides contenant les acides gras insaturés et le fait de permettre aux lipides et aux acides insaturés d'être absorbés par les particules de coeur ; et
- la récupération du granulé.

15. Procédé selon la revendication 14, dans lequel les particules de coeur sont préparées en agglomérant une poudre comprenant le métal pro-oxydant et facultativement d'autres ingrédients pulvérulents, en pulvérisant la poudre avec un liquide aqueux.

16. Procédé selon la revendication 15, dans lequel la poudre est pulvérisée avec une solution aqueuse d'un saccharide choisi dans le groupe constitué par les monosaccharides, les disaccharides et leurs combinaisons, puis un séchage est réalisé pour obtenir la poudre agglomérée.

17. Procédé selon l'une quelconque des revendications 14 à 16, dans lequel les particules de coeur sont pulvérisées avec les lipides à une température inférieure à 50 °C, de préférence inférieure à 40 °C.

18. Utilisation d'un granulé selon l'une quelconque des revendications 1 à 13, dans la préparation d'une composition utilisable dans le traitement ou la prévention d'un développement mental retardé ou d'une fonction cognitive diminuée.
